# EUROPEAN PATENT SPECIFICATION

(11) **EP 1 951 229 B1**
(45) Date of publication and mention of the grant of the patent: **10.07.2013**
(21) Application number: 06809871.4
(22) Date of filing: 14.11.2006
(51) Int. Cl.: A61K 31/4015, A61P 27/06

(54) **SUCCINIMIDE DERIVATIVES AS OCULAR HYPOTENSIVE AGENTS**
SUCCINIMID-DERIVATE ALS OKULARE HYPOTENSIVE MITTEL
DERIVES DE SUCCINIMIDE EN TANT QU'AGENTS HYPOTENSIFS OCULAIRES

(30) Priority: 21.11.2005 IL 17207005; 28.11.2005 US 739964 P
(43) Date of publication of application: 06.08.2008
(73) Proprietor: Sharir, Mordechai, 75463 Rishon Lezion (IL)
(72) Inventor: Sharir, Mordechai, 75463 Rishon Lezion (IL)
(74) Representative: Grünecker, Kinkeldey, Stockmair & Schwanhäusser
(86) International application number: PCT/IL2006/001315
(87) International publication number: WO 2007/057889

(56) References cited:
- US-A- 4 188 398
- US-A- 4 609 663
- US-A- 4 981 867
- US-A1- 2005 175 690

## Description

### FIELD OF THE INVENTION

The present invention generally relates to ocular disorders and more specifically to the use of anti-epileptic succinimide derivatives for treating ocular disorders associated with elevated intraocular pressure, such as glaucomas.

### BACKGROUND OF THE INVENTION

The present invention focuses on the similarities in the pathophysiology of *Petit Mal* (Absence) epilepsy and the glaucomas. Both diseases reflect a change in rate of secretion and/or defective outflow facility, which creates a local tissue electrolytic, ionic and osmotic imbalance, producing a characteristic cascade of symptoms.

Glaucomas are a family of ocular disorders usually characterized by an increased intraocular pressure (IOP) with a typical damage to the optic nerve and the visual field, but many exceptions exist. The level of IOP is the net result of production minus outflow from the eye, via a ring-like sieve structure called the trabecular meshwork, located at the angle of the anterior chamber. While most of the hypertensive glaucomas result from increased trabecular resistance to outflow, most of the medical therapy focuses on decreasing the inflow (See Shields M.B.: Textbook of Glaucoma, 4th Ed., Williams & Wilkins, Baltimore, 1998). Every minute approximately 1.8-4.2 microliter of aqueous humor is produced and secreted into the posterior chamber of the eye by the non-pigmented ciliary epithelium. The process is not well understood, but seems to involve a combination of active ultrafiltration and passive transport. The rate of secretion is influenced by multiple factors, e.g. diurnal curve, pH, age, enzymes like carbonic anhydrase (CA) as well as vascular diseases. Several anti-glaucoma drug classes influence various stages of the aqueous humor flow, e.g. beta-adrenoreceptor antagonists (timolol, betaxolol,) systemic and topical CA inhibitors (acetazolamide, dorzolamide, See Sharir M.: Novel Thiadiazole Sulfonamide Carbonic Anhydrase Inhibitors as Topically Effective Ocular Hypotensive Agents, PhD Thesis, University of Louisville, Louisville, KY, USA, May 1990), alpha-adrenoreceptor agonists (brimonidine, apraclonidine) and prostaglandin analogues (latanoprost, bimatoprost) to name a few. Most drug groups act synergistically to decrease aqueous humor production and secretion by up to 50% with some 'unconventional' outflow additive effect of the prostaglandins. Studies in aqueous humor dynamics elicited some pivotal components in the secretion processes. While bicarbonate is found in higher than plasma concentration in the posterior chamber of the rabbit (probably a direct result of CA involvement), it is not the case in humans, where chloride has been suggested as the key anion.

The mechanism of fluid secretion across semi-permeable/selective tissue membranes in the human body shares similarities in most organ systems. At some point, in the cell membrane, "water splitting" occurs: the proton follows an anion (to maintain electro-neutrality) and gets to one side while the hydroxyl usually couples with sodium or another positively charged component and ends up in the contra-lateral side of the cell membrane. The substrate used for this water splitting is carbon dioxide, which gets hydrated to form the (weak) carbonic acid; consequently its proton and bicarbonate are separated by the cell membrane. This process generates passive water secretion, to accompany the electrolytes and maintain both electric and osmotic equilibrium. The reaction is catalyzed by CA.

Aqueous humor is produced and secreted into the posterior chamber of the eye by the non-pigmented ciliary epithelium (glaucoma), similarly to the cerebrospinal fluid (CSF), formed by the choroidal plexus and secreted across the floor of the brain ventricles. A basolateral Chloride/Bicarbonate-anion exchanger switches between the two and it is suggested that succinimides, by way of disrupting first the T-calcium channels and then the anion equilibrium, disrupt aqueous humor production, hence decreasing the intraocular pressure.

The typical absence epilepsy of childhood is a non-convulsive form of epilepsy that is characterized by frequent "absences" and bilaterally synchronous 3/s spike and wave electroencephalographic features, often called 'spike-wave-discharge' (SWD). Absence seizures are idiopathic and are divided according to the age of onset to childhood absence epilepsy (CAE, or pyknolepsy), juvenile absence epilepsy (JAE) and juvenile myoclonic epilepsy (JME or impulsive *Petit Mal* seizures). All these conditions are associated with the SWD pattern, and seizures that may last from few seconds to minutes, sometimes several hundred attacks per day. The pathogenesis is still unknown. Multiple studies suggest that epilepsy can result from processes which disturb extracellular ion homeostasis, alter energy metabolism, change receptor function or alter transmitter uptake. Recent studies suggest that a reverberant thalamo-cortical neuronal circuitry underlies the SWD seizures. (See Huguenard J.R.: Neuronal Circuitry of Thalamocortical Epilepsy and Mechanism of Anti-absence Drug Action. In: Jasper's Basic Mechanism of the Epilepsies, 3rd Ed., Advances in Neurology, Vol. 79, Chapter 67, edited by A.V. Delgado-Escueta et. al. Lippincott Williams & Wilkins, Philadelphia, 1999). Ethosuximide and its methsuximide metabolite may exert their action through alteration in thalamic cellular excitability, possibly by blocking the T-type calcium current, while a tetramethyl derivative might cause convulsions (Coulter D.A., Huguenard J.R., Prince D.A.: Characterization of ethosuximide reduction of low-threshold calcium current in thalamic neurons. Ann. Neurol. 1989;25:582-593.). While T channel blockade is important, it is not the sole anti-absence drug mechanism. The effectiveness of the benzodiazepine clonazepam in ameliorating absence epilepsy suggested that the GABA receptor system is important as well as a wider network of neuronal system. Other theories suggest that some of the CAE or JAE, especially the hereditary types are because of defects in CLCN-2 (Chloride channel Protein 2).

Succinimide derivatives for use in medicine are also disclosed in US 4,188,398; US 4,981,867; US 4,609,663 and US 2005/175690A1.

### SUMMARY OF THE PRESENT INVENTION

The present invention provides methods and pharmaceutical compositions for treating ocular disorders associated with elevated intraocular pressure, such as glaucoma, which are based on a novel use of anti-epileptic compounds of the succinimide family as ocular hypotensive agents.

According to the invention there is provided a method for treating glaucomas by administrating to a subject in need of such a treatment an effective amount of a pharmaceutical composition containing a succinimide derivative as the active ingredient. Preferably, the succinimide derivative is selected from the group consisting of ethosuximide, methsuximide, phensuximide, and morsuximide, or an anti-epileptic compound of structure I : 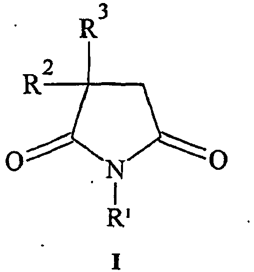 where R¹ is selected from the group consisting of H, alkyl, alkylaryl, alkyl-heteroaryl, alkyl-cycloalky and alkyl-cycloheteroalkyl; and R² is H, R³ is selected from the group consisting of H, aryl, and aryloxyalkyl.
or of structure II: 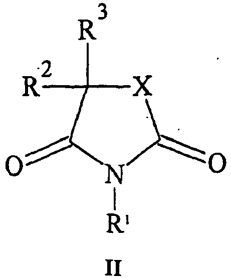 where X is -O- or -CHR⁴, R¹ is selected from the group consisting of H, alkyl, alkylaryl, alkyl- heteroaryl, alkyl-cycloalkyl and alkyl-cycloheteroalkyl; and R² is H, and R³ and R⁴ are independently selected from the group consisting of H, aryl, and aryloxyalkyl. When X is an oxygen atom, the compounds are known as oxazolidinedione and oxazolidinedione derivatives.

Preferably the pharmaceutical composition is administrated topically to the eye of the subject. However, administration can be local or systemic.

Another aspect of the invention is a topical ophthalmic pharmaceutical composition for the treatment of glaucoma comprising a succinimide derivative as an active agent and a carrier suitable for topical delivery. The topical pharmaceutical compositions of the invention may be formulated as solutions, suspensions, gels and emulsions to be applied as eye-drops or as ointments, and may contain, besides the active ingredient and the carrier, other pharmaceutically acceptable agents and excipients, such as such as stabilizers, preservatives, chelating agents, viscosity modifying agents, buffering agents and/or pH adjusting agents. Additionally, the compositions may contain other ophthalmic active agents such as antibacterial agents, comfort enhancers, antioxidants and the like The compositions may further contain controlled release means.

A further aspect of the invention is the use of a succinimide derivative for the treatment of ocular disorders associated with ocular hypertension and their use in the manufacture of an ophthalmic pharmaceutical composition for the treatment of ocular disorders associated with ocular hypertension.

### DESCRIPTION OF THE INVENTION

The succinimide ring continues to be incorporated into many new compounds with diverse applications. In the pharmaceutical field alone, succinimide-derived drugs have recently been evaluated as anti-tumor agents, oxytocin antagonists, anti-HIV drugs, anti-anxiety agents, analgesics and aldose reductase inhibitors (Rankin G.O.: Nephrotoxicity induced by C- and N-Arylsuccinimides. J. Toxicol. Envirom. Health, Part B, 7:399-416, 2004). The present invention focuses on the anti-glaucoma properties of succinimides, in particular on succinimides that exhibit antiepileptic and/or antiseisure activity and more particularly on succinimides that are useful for absence seizures, such as ethosuximide, phensuximide, methosuximide and morsuximide. However the invention is not limited to the particular succinimides listed above or to succinimides that already have been found to have anti-epileptic activity but encompasses new anti-epileptic or other succinimide-derived drugs yet to be found, as well. In accordance with the present invention, it is proposed that the antiepileptic medications of the succinimide type may have ocular hypotensive efficacy by modulating the ionic channels, controlling the rate of production and secretion of the aqueous humor from the non-pigmented ciliary body of the eye. Accordingly, the present invention provides a novel use of anti-epileptic drugs, and in particular of anti-epileptic compounds which belong to the succinimide family, for the treatment of ocular disorders associated with elevated ocular pressure and of glaucomas in particular.

Preferably, the succinimide-derived compounds of the invention are of formula I: 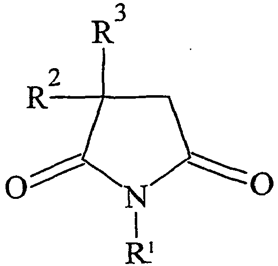 where R¹ is selected from the group consisting of H, alkyl, alkylaryl, alkyl-heteroaryl, alkyl-cycloalky and alkyl-cycloheteroalkyl; and R² is H, R³ is selected from the group consisting of H, aryl, and aryloxyalkyl.
or of formula II: 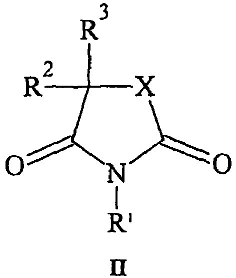 where X is -O- or -CHR⁴, R¹ is selected from the group consisting of H, alkyl, alkylaryl, alkyl- heteroaryl, alkyl-cycloalkyl and alkyl-cycloheteroalkyl; and R² is H, R³ and R⁴ are independently selected from the group consisting of H, aryl, and aryloxyalkyl. When X is an oxygen atom, the compounds are known as oxazolidinedione and oxazolidinedione derivatives.

Specific examples of succinimide derivatives of structure I already in use as antiepliptic drugs are the ethosuximide (R¹ = H, R² = ethyl, R³ = methyl), phensuximide (R¹ = methyl, R² = H, R³ = phenyl); methosuximide (R¹ = methyl, R² = methyl, R³ = phenyl); and morsuximide (R¹ = methylmorpholine, R² = H, R³ = phenyl). Other succinimide derivatives of structure I were also found to exhibit antiseizure activity. For example, US patent No. 4,188,398 teaches antiepileptic activity of α-/para-isopropyloxyphenil/succinimide (R¹ =H, R² = H, R³ = phenyloxyisopropyl). US patent No. 4,981,867 discloses the use of succinimides of structure II for reducing tremor. Yet, the invention is not limited to those particular succinimide derivatives that already have been proved to exhibit antieplileptic or antiseizure activity.

Thus, the present invention provides succinimide derivatives for use in a method for treating disorders associated with elevated intraocular pressure, and in particular for treating glaucoma, by administrating an effective amount of pharmaceutical compositions comprising an anti-epileptic succinimide-derived compound as the active ingredient and a pharmaceutically acceptable carrier.

Preferably, the pharmaceutical compositions of the invention are administrated topically onto the eye of a patient for facilitating effective intraocular levels of the drug and for preventing unnecessary drug level in other organs. Such a non-systemic, site-specific administration reduces the side effects associated with the drugs. However, oral or otherwise systemic administration in a dosage effective for reducing the intraocular pressure is also possible. For example, the composition may be administrated by a dermal patch for extended release.

When administration is topical, the pharmaceutical compositions containing the succinimide derivative may be formulated in various therapeutic forms suitable for topical delivery, including solutions, suspensions, emulsions and gels. The carrier in these formulations may be any pharmaceutical acceptable carrier such as saline, buffered saline, carbopol gel, mineral oil and the like. The formulations can be prepared in accordance with known procedures for the preparation of ophthalmic formulations. Preferably, the concentration of the succinimide derivative in the pharmaceutical compositions is in the range of 50 to 2500 mg/ml and the formulation is preferably applied one to four doses wherein each dose contains 10 to 500 mg of succinimide derivative.

The topical pharmaceutical compositions may be in the form of eye-drops to be applied by instillation into the eye or may be in the form of a viscous ointment, gel or cream to be applied by an ointment onto the ocular surface and may contain control release means for facilitating sustained release over a prolong period of time.

The compositions may further include non-toxic auxiliary pharmaceutically acceptable substances such as stabilizers, preservatives, chelating agents, viscosity modifying agents, buffering agents and/or pH adjusting agents. Additionally, the compositions may contain other ophthalmic active agents such as antibacterial agents, comfort enhancers, antioxidants and the like.

In accordance with other embodiments, the succinimide derivative may be loaded into a drug-delivery device to be inserted or implanted into the eye of the patient for allowing releasing of the drug in a controlled and continuous rate, by dissolving, diffusion or leaching, thus maintaining effective therapeutic concentration over a prolonged period of time. The drug-delivery device may be for example a biocompatible thin film loaded with the active agent, inserted for example beneath the lower eyelid.

### EXPERIMENTAL

Preliminary ocular pharmacodynamic studies were conducted at the Tel-Aviv University animal facility to study the effect of ethosuximide on the intraocular pressure in rats by measuring IOP before and after administration of ethosuximide. IOP measurements were taken by a tonopen instrument. Ethosuximide was applied as a viscous solution obtained from Petnidan® capsules (Desitin, Hamburg, Germany). A recent study at Alcon Research laboratory (Pang IH, Wang WH, Clark AF: Acute effects of glaucoma medications on rat intraocular pressure, Exp Eye Res 2005, Feb; 80(2)207-14) has shown rats to be a proper animal model for glaucoma study. Another study in the Casey Eye Institute (Moore CG, Milne ST, Morrison JC: Noninvasive measurements of rat intraocular pressure with the Tono-Pen. Invest. Ophthalmol. Vis. Sci. 1993 Feb;34(2)363-9) has shown that a tonopen can be used reliably to measure IOP in normal rat eye.

Six Dark Agauti (DA) pigmented rats, (250-300g in weight) were slightly sedated with 1.5-2mg (0.15-0.2 ml) of intraperitoneal xylazine. One eye of each rat was randomly selected to receive a topical administration of 50 µl of 250mg/0.2ml ethosuximide solution (content of Petnidan capsules). The second (control) eye received 50 µl of viscoelastic solution without the active ingredient (Viscotears™ manufactured by Novartis, Switzerland). At t=0 (immediately before) and at 30, 60 and 120 minutes after administration, topical anesthetic benoxinate was applied to both eyes and the intraocular pressures (IOP) were measured by a Tono-Pen XL tonometer (Medtronics). IOP values were recorded as the mean value of 6-10 successive measurements to minimize variability. The code, namely which eye of a pair was treated, was unknown to the technician who performed the IOP measurements. One rat expired after 1 hour due to excessive sedation. Five rats completed the experiment. In 2 out of 6 eyes that received the drug (vs. 1 of 6 eyes that were administrated viscoelastic vehicle) a mild to moderate limbal vascular congestion was noticed, which tended to subside at 60 minutes.

At the end of the experiment, the code was broken and the IOP values in control and treated eyes were compared by statistical analysis. The results are summarized in following Table 1.

**Table 1: IOP values (mmHg) measured in rats before (t=0) and at 30, 60 and 120 minutes after administration of ethosuximide solution: T=treated eye; or vehicle only: C= control eye.**

| Time | 0 | | 30 min | | 60 min | | 120 min | |
|---|---|---|---|---|---|---|---|---|
| eye | C | T | C | T | C | T | C | T |
| Rat #1 | 15 | 15.5 | 13 | 17 | 20.3 | 21.3 | 32.5 | 22 |
| Rat #2 | 13 | 17 | 13.5 | 16 | 30 | 30 | 17 | 17 |
| Rat #3 | 22 | 22 | 19 | 15 | 20.3 | 18 | 20.5 | 19 |
| Rat #4 | 23 | 25 | 19 | 15.3 | 16 | 14.6 | 20 | 20.5 |
| Rat #5 | 15 | 18 | 18 | 20.5 | 20.3 | 21.3 | 19.7 | 20.2 |
| Rat #6 | 20 | 24 | 26 | 22 | - | - | - | - |
| Total | 108 | 121.5 | 108.5 | 105.8 | 106.9 | 105.2 | 111.6 | 98.7 |
| Average *X̅* | 18 | 20.2 | 18.1 | 17.6 | 21.4 | 21.0 | 22.3 | 19.7 |
| Δ (IOP) C-T | -2.2 | | +0.5 (p > 0.05) | | +0.4 (p > 0.05) | | +2.6 (p < 0.05) | |

As can be seen in Table 1, at 30 and 60 minutes after administration, a modest decrease in the IOP was depicted, (0.5 mmHg and 0.4 mmHg respectively), which was not significant either clinically or statistically. However, at 120 minutes, a decrease of 2.6 mmHg was noticed in the treated eyes versus control. This clinically significant effect was also statistically significant (p < 0.05, using two-tailed Student's t-test for paired data). It was also statistically significant when using ANOVA analysis.

The results of the preliminary study clearly indicate the potential of ethosuximide and other succinimide derivatives as ocular hypotensive agents and their use in the preparation of anti glaucoma drugs, in particular in the preparation of ophthalmic pharmaceutical compositions for topical administration.

## Claims

1. A succinimide derivative selected from the group consisting of ethosuximide, methsuximide, phensuximide and morsuximide, or a succinimide derivative of the formula: 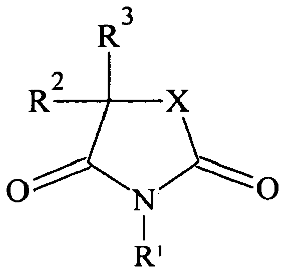 where R¹ is selected from the group consisting of H, alkyl, alkylaryl, alkyl-heteroaryl, alkyl-cycloalkyl and alkyl-cycloheteroalkyl;
X is -O- or -CHR⁴; R² is H;
R³ and R⁴ are independently selected from the group consisting of H, aryl and aryloxyalkyl,
for use in a method of treating ocular disorders associated with ocular hypertension.

2. The succinimide derivative for use according to claim 1, wherein said ocular disorder is glaucoma.

3. The succinimide derivative for use according to claim 1, wherein said succinimide derivative is an anti-epileptic agent.

4. The succinimide derivative for use according to claim 3, wherein said anti-epileptic agent is active against absence seizures.

5. The succinimide derivative for use according to any of claims 1 to 4, wherein said pharmaceutical composition is to be applied topically to the eye of said subject.

6. The succinimide derivative for use according to claim 5, wherein the succinimide derivative is to be applied in 1 to 4 doses a day wherein each dose contains 10 to 500 mg of the succinimide derivative.

7. The succinimide derivative for use according to any of claims 1 to 4, wherein said succinimide derivative is administrated by means of a drug-delivery device inserted into the eye of said subject.

8. A topical ophthalmic pharmaceutical composition for use in a method of treating an ocular disorder associated with ocular hypertension, the composition comprising a succinimide derivative selected from the group consisting of ethosuximide, methsuximide, phensuximide and morsuximide, or a succinimide derivative of the formula: 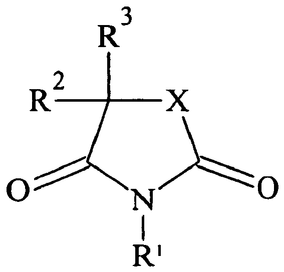 where R¹ is selected from the group consisting of H, alkyl, alkylaryl, alkyl-heteroaryl, alkyl-cycloalkyl and alkyl-cycloheteroalkyl;
X is -O- or -CHR⁴;
R² is H; and
R³ and R⁴ are independently selected from the group consisting of H, , aryl, and aryloxyalkyl; and
a pharmaceutically acceptable carrier for topical application to the eye.

9. The composition for use according to claim 8 in the form selected from an eye-drops solution, an ointment, a suspension, a gel or a cream.

10. The composition for use according to claim 8, wherein the succinimide derivative is an anti-epileptic agent.

11. The composition for use according to claim 8, wherein the concentration of the succinimide derivative is in the range of 50 to 2500 mg/ml.

12. The composition for use according to claim 8, further comprising at least one pharmaceutically acceptable agent, preferably one or more of the following: a stabilizer, a preservative, a chelating agent, a viscosity modifying agent, a buffering agent or pH adjusting agent.

13. The composition for use according to claim 8, further comprising an additional ophthalmic active agent, preferably an antibacterial agent, a comfort enhancer, or an antioxidant.

14. The composition for use according to claim 8, wherein the ocular disorder is glaucoma.

15. Use of a succinimide derivative selected from the group consisting of ethosuximide, methsuximide, phensuximide and morsuximide, or a succinimide derivative of the formula: 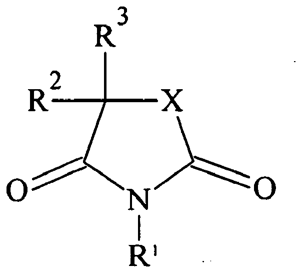 where R¹ is selected from the group consisting of H, alkyl, alkylaryl, alkyl-heteroaryl, alkyl-cycloalkyl and alkyl-cycloheteroalkyl;
X is -O- or -CHR⁴; R² is selected H; and
R³ and R⁴ are independently selected from the group consisting of H, aryl, and aryloxyalkyl,
for the manufacture of a medicament for the treatment of ocular disorders associated with ocular hypertension.

16. The use according to claim 15, wherein said ocular disorder is glaucoma.

17. The use according to claim 15 or 16, wherein the medicament is to be applied topically to the eye of said subject, or by means of a drug-delivery device inserted into the eye of said subject.

18. Use of a topical ophthalmic pharmaceutical composition according to any one of claims 8-13 for the preparation of a medicament for use in a method of treating an ocular disorder associated with ocular hypertension.

19. The use according to claim 18, wherein said ocular disorder is glaucoma.

## Patentansprüche

1. Ein Succinimid-Derivat, ausgewählt aus der Gruppe bestehend aus Ethosuximid, Methsuximid, Phensuximid und Morsuximid, oder ein Succinimid-Derivat der Formel: 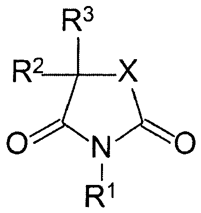 wobei R¹ ausgewählt ist aus der Gruppe bestehend aus H, Alkyl, Alkylaryl, Alkyl-Heteroaryl, Alkyl-Cycloalkyl und Alkyl-Cycloheteroalkyl;
X -O- oder -CHR⁴ ist; R² H ist;
R³ und R⁴ unabhängig voneinander ausgewählt sind aus der Gruppe bestehend aus H, Aryl und Aryloxyalkyl,
zur Verwendung in einem Verfahren zur Behandlung okulärer Störungen, die mit okulärer Hypertension assoziiert sind.

2. Das Succinimid-Derivat zur Verwendung gemäß Anspruch 1, wobei die genannte okuläre Störung Glaukom ist.

3. Das Succinimid-Derivat zur Verwendung gemäß Anspruch 1, wobei das genannte Succinimid-Derivat ein Antiepileptikum ist.

4. Das Succinimid-Derivat zur Verwendung gemäß Anspruch 3, wobei das genannte Antiepileptikum gegenüber Petit-mal (absence seizures) wirksam ist.

5. Das Succinimid-Derivat zur Verwendung gemäß einem beliebigen der Ansprüche 1 bis 4, wobei die genannte pharmazeutische Zusammensetzung topisch auf das Auge des genannten Subjekts anzuwenden ist.

6. Das Succinimid-Derivat zur Verwendung gemäß Anspruch 5, wobei das Succinimid-Derivat in 1 bis 4 Dosen pro Tag zu verabreichen ist, wobei eine jede Dosis 10 bis 500 mg des Succinimid-Derivats enthält.

7. Das Succinimid-Derivat zur Verwendung gemäß einem beliebigen der Ansprüche 1 bis 4, wobei das genannte Succinimid-Derivat mittels einer Medikamenten-Verabreichungsvorrichtung, die in das Auge des Subjekts eingebracht wurde, verabreicht wird.

8. Eine topische, ophthalmische, pharmazeutische Zusammensetzung zur Verwendung in einem Verfahren zur Behandlung einer okulären Störung, die mit okulärer Hypertension assoziiert ist, die Zusammensetzung umfassend ein Succinimid-Derivat ausgewählt aus der Gruppe bestehend aus Ethosuximid, Methsuximid, Phensuximid und Morsuximid, oder ein Succinimid-Derivat der Formel: 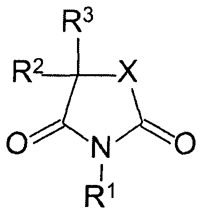 wobei R¹ ausgewählt ist aus der Gruppe bestehend aus H, Alkyl, Alkylaryl, Alkyl-Heteroaryl, Alkyl-Cycloalkyl und Alkyl-Cycloheteroalkyl;
X -O- oder -CHR⁴ ist;
R² H ist; und
R³ und R⁴ unabhängig voneinander ausgewählt sind aus der Gruppe bestehend aus H, Aryl und Aryloxyalkyl; und
einen pharmazeutisch verträglichen Träger zur topischen Verabreichung in das Auge.

9. Die Zusammensetzung zur Verwendung gemäß Anspruch 8 in der Form ausgewählt aus der Gruppe bestehend aus einer Augentropfenlösung, einer Salbe, einer Suspension, einem Gel oder einer Creme.

10. Die Zusammensetzung zur Verwendung gemäß Anspruch 8, wobei das Succinimid-Derivat ein Antiepileptikum ist.

11. Die Zusammensetzung zur Verwendung gemäß Anspruch 8, wobei die Konzentration des Succinimid-Derivats im Bereich von 50 bis 2500 mg/ml liegt.

12. Die Zusammensetzung zur Verwendung gemäß Anspruch 8, ferner umfassend wenigstens ein pharmazeutisch verträgliches Agens, vorzugsweise eines oder mehrere der folgenden: einen Stabilisator, ein Konservierungsmittel, einen Chelatbildner, ein viskositätsmodifizierendes Agens,
eine Puffersubstanz oder ein pH-regulierendes Agens.

13. Die Zusammensetzung zur Verwendung gemäß Anspruch 8, ferner umfassend ein zusätzliches ophthalmisch aktives Agens, vorzugsweise ein antibakterielles Agens, einen Komfortverbesserer, oder ein Antioxidans.

14. Die Zusammensetzung zur Verwendung gemäß Anspruch 8, wobei die okuläre Störung Glaukom ist.

15. Verwendung eines Succinimid-Derivats, ausgewählt aus der Gruppe bestehend aus Ethosuximid, Methsuximid, Phensuximid und Morsuximid, oder ein Succinimid-Derivat der Formel: 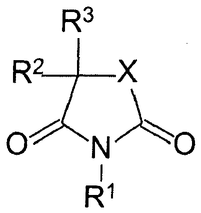 wobei R¹ ausgewählt ist aus der Gruppe bestehend aus H, Alkyl, Alkylaryl, Alkyl-Heteroaryl, Alkyl-Cycloalkyl und Alkyl-Cycloheteroalkyl;
X -O- oder -CHR⁴ ist; R² als H ausgewählt ist; und
R³ und R⁴ unabhängig voneinander ausgewählt sind aus der Gruppe bestehend aus H, Aryl und Aryloxyalkyl,
zur Herstellung eines Medikaments zur Behandlung von okulären Störungen, die mit okulärer Hypertension assoziiert sind.

16. Die Verwendung gemäß Anspruch 15, wobei die genannte okuläre Störung Glaukom ist.

17. Die Verwendung gemäß Anspruch 15 oder 16, wobei das Medikament topisch auf das Auge, oder mittels einer Medikamenten-Verabreichungsvorrichtung, die in das Auge des genannten Subjekts eingebracht wurde, anzuwenden ist.

18. Verwendung einer topischen, ophthalmischen, pharmazeutischen Zusammensetzung gemäß einem beliebigen der Ansprüche 8-13 zur Herstellung eines Medikaments zur Verwendung in einem Verfahren zur Behandlung einer okulären Störung, die mit okulärer Hypertension assoziiert ist.

19. Die Verwendung gemäß Anspruch 18, wobei die genannte okuläre Störung Glaukom ist.

## Revendications

1. Dérivé de succinimide sélectionné dans le groupe constitué par l'éthosuximide, le methsuximide, le phensuximide et le morsuximide, ou un dérivé de succinimide de formule : 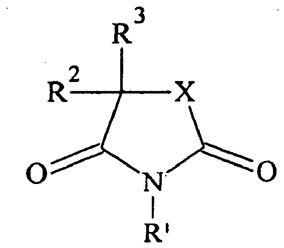 où R¹ est sélectionné dans le groupe constitué par H et les groupes alkyle, alkylaryle, alkyl-hétéroaryle, alkyl-cycloalkyle et alkyl-cyclohétéroalkyle ;
X est -O- ou -CHR⁴ ; R² est H ;
R³ et R⁴ sont indépendamment sélectionnés dans le groupe constitué par H et les groupes aryle et aryloxyalkyle,
destiné à être utilisé dans une méthode de traitement des troubles oculaires associés à l'hypertension oculaire.

2. Dérivé de succinimide destiné à être utilisé selon la revendication 1, ledit trouble oculaire étant le glaucome.

3. Dérivé de succinimide destiné à être utilisé selon la revendication 1, lequel dérivé de succinimide est un agent anti-épileptique.

4. Dérivé de succinimide destiné à être utilisé selon la revendication 3, ledit agent anti-épileptique étant actif contre les crises d'absence.

5. Dérivé de succinimide destiné à être utilisé selon n'importe lesquelles des revendications 1 à 4, ladite composition pharmaceutique devant être appliquée par voie topique sur l'oeil dudit sujet.

6. Dérivé de succinimide destiné à être utilisé selon la revendication 5, lequel dérivé de succinimide doit être appliqué en 1 à 4 doses par jour, chaque dose contenant 10 à 500 mg du dérivé de succinimide.

7. Dérivé de succinimide destiné à être utilisé selon n'importe lesquelles des revendications 1 à 4, lequel dérivé de succinimide doit être administré au moyen d'un dispositif d'administration de médicament introduit dans l'oeil dudit sujet.

8. Composition pharmaceutique ophtalmique topique destinée à être utilisée dans une méthode de traitement d'un trouble oculaire associé à l'hypertension oculaire, la composition comprenant un dérivé de succinimide sélectionné dans le groupe constitué par l'éthosuximide, le methsuximide, le phensuximide et le morsuximide, ou un dérivé de succinimide de formule : 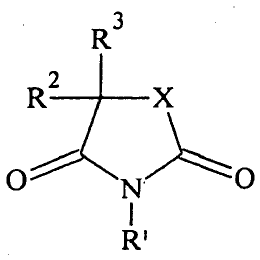 où R¹ est sélectionné dans le groupe constitué par H et les groupes alkyle, alkylaryle, alkyl-hétéroaryle, alkyl-cycloalkyle et alkyl-cyclohétéroalkyle ;
X est -O- ou -CHR⁴ ;
R² est H ; et
R³ et R⁴ sont indépendamment sélectionnés dans le groupe constitué par H et les groupes aryle et aryloxyalkyle ; et
un véhicule pharmaceutiquement acceptable pour une application topique sur l'oeil.

9. Composition destinée à être utilisée selon la revendication 8, sous une forme sélectionnée parmi une solution de gouttes oculaires, une pommade, une suspension, un gel ou une crème.

10. Composition destinée à être utilisée selon la revendication 8, dans laquelle le dérivé de succinimide est un agent anti-épileptique.

11. Composition destinée à être utilisée selon la revendication 8, dans laquelle la concentration du dérivé de succinimide se situe dans la plage de 50 à 2500 mg/ml.

12. Composition destinée à être utilisée selon la revendication 8, comprenant en outre au moins un agent pharmaceutiquement acceptable, de préférence un ou plusieurs parmi les suivants : stabilisant, conservateur, agent chélatant, agent modificateur de viscosité, agent tampon ou agent d'ajustement du pH.

13. Composition destinée à être utilisée selon la revendication 8, comprenant en outre un agent actif ophtalmique supplémentaire, de préférence un agent antibactérien, un agent améliorant le confort, ou un antioxydant.

14. Composition destinée à être utilisée selon la revendication 8, le trouble oculaire étant le glaucome.

15. Utilisation d'un dérivé de succinimide sélectionné dans le groupe constitué par l'éthosuximide, le methsuximide, le phensuximide et le morsuximide, ou un dérivé de succinimide de formule : 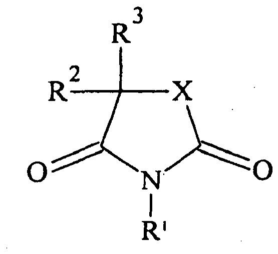 où R¹ est sélectionné dans le groupe constitué par H et les groupes alkyle, alkylaryle, alkyl-hétéroaryle, alkyl-cycloalkyle et alkyl-cyclohétéroalkyle ;
X est -O- ou -CHR⁴ ; R² est choisi H ; et
R³ et R⁴ sont indépendamment sélectionnés dans le groupe constitué par H et les groupes aryle et aryloxyalkyle,
pour la fabrication d'un médicament destiné au traitement des troubles oculaires associés à l'hypertension oculaire.

16. Utilisation selon la revendication 15, ledit trouble oculaire étant le glaucome.

17. Utilisation selon la revendication 15 ou 16, dans laquelle le médicament doit être appliqué par voie topique sur l'oeil dudit sujet, ou au moyen d'un dispositif d'administration de médicament introduit dans l'oeil dudit sujet.

18. Utilisation d'une composition pharmaceutique ophtalmique topique selon l'une quelconque des revendications 8 à 13 pour la préparation d'un médicament destiné à être utilisé dans une méthode de traitement d'un trouble oculaire associé à l'hypertension oculaire.

19. Utilisation selon la revendication 18, ledit trouble oculaire étant le glaucome.
